# EUROPEAN PATENT SPECIFICATION

(11) **EP 2 144 587 B1**
(45) Date of publication and mention of the grant of the patent: **12.10.2016**
(21) Application number: 08767456.0
(22) Date of filing: 30.04.2008
(51) Int. Cl.: A61H 33/06, A61L 9/03, A61L 9/04, A01M 1/20

(54) **VAPOR-EMITTING DEVICE WITH END-OF-USE INDICATOR**
DAMPFEMISSIONSVORRICHTUNG MIT ANZEIGE DES VERWENDUNGSENDES
DISPOSITIF D'ÉMISSION DE VAPEUR AVEC INDICATEUR DE FIN D'UTILISATION

(30) Priority: 03.05.2007 US 799913
(43) Date of publication of application: 20.01.2010
(73) Proprietor: The Dial Corporation, Scottsdale, AZ 85260 (US)
(72) Inventor: BANKERS, Jeffrey, Phoenix, AZ 85041 (US); BUTTERWORTH, Debra, K., Scottsdale, AZ 85257 (US); FAIRES, Geoffrey, Cave Creek, AZ 85331 (US)
(74) Representative: Henkel IP Department
(86) International application number: PCT/US2008/005550
(87) International publication number: WO 2008/136993

(56) References cited:
- EP-A2- 0 495 631
- GB-A- 2 372 449
- US-A- 5 647 052
- US-A1- 2007 058 955
- US-A1- 2007 058 956
- US-B1- 6 368 564
- US-B1- 6 368 564
- US-B2- 6 920 282

## Description

### FIELD OF THE INVENTION

The present invention relates to vapor-emitting devices, and more particularly to a gel-based vapor-emitting device that includes an end-of-use indicator physically anchored within the evaporating mass.

### BACKGROUND OF THE INVENTION

Vapor-emitting devices are generally used to volatize air treatment materials such as fragrances, air sanitizers, insecticides or insect repellants or other vaporizable materials into a surrounding environment. A common type of vapor-emitting device operates by passive evaporation of a fragrance or other volatile material from a solid gel matrix exposed to an environment to be treated. Air treatment occurs through evaporation of the air treatment materials over an extended time period that is sometimes referred to as the "length-of-life" of the product. The vapor-emitting device may provide, amongst other things, a pleasing fragrance or an odor neutralizer over a useful period of time, such as 30-days. During use, the material within the vapor-emitting device gradually disappears until the time is reached to replace or refill the device (the "end-of-life", or "end-of-use" period). For example, in liquid vaporizers the liquid level in a bottle or larger vessel may be easily observed to decrease over time, and that point when the liquid is entirely gone may also be an easily observed indication of the need to replace or refill the product. In a gel-matrix based device, the starting physical mass of gel is seen to shrink and shrivel up over time as the volatiles such as water, other solvents and the air treatment material(s), evaporate. In order to judge when it's time to replace the vapor-emitting device, or to refill it if possible, users visually inspect and/or smell the remaining contents of the vapor-emitting device. However, the user must have the capability of determining that the vapor-emitting device is nearing depletion of the volatizable air-treatment ingredient(s) and that it is no longer capable of emitting a useful vapor. This is particularly problematic for gel-based products since some physical mass will remain even though the volatile constituents are all but gone. Since gel-based vapor-emitting products are made using polymeric gelling agents (e.g., carrageenans, or other polysaccharide/cellulosic, or polyacrylic gellants), there are non-volatile components that simply won't disappear over time. A classic example of such a product is the gel-based air freshener Renuzit® Adjustables® that dries up over about 30-days into a shrunken and dried mass of left over polysaccharidic polymer. As a consequence of having a remaining mass after the usable length-of-life, consumers may not replace their gel-based vapor-emitting device because they believe there is some useful product still remaining, even though the dried up and shrunk mass that remains is entirely useless. Certainly there is a strong business reason to ensure regular "repurchase rate" by consumers. In order to achieve a predictable repurchase rate, marketers must ensure that the consumer does not extend a product beyond its useful life. For example, it is undesirable to have consumers leave an air freshener in their home for months, ignoring that the label claims a shorter life, e.g., "Lasts up to 30-Days".

As discussed, many current vapor-emitting devices, and in particular gel-based air treatment products, do not include a means for visually alerting the user that the vapor-emitting device is low on useful volatizable ingredient(s) and thus no longer capable of producing air treatment vapor, (i.e., at the end of its "length-of-life" and at time for repurchase). EP 495 631 A2 discloses a gel-based vapor-emitting device that includes an end-of-use indicator.

### BRIEF SUMMARY OF THE INVENTION

It should thus be appreciated that it is very desirable to provide an alternative gel-based vapor-emitting device that includes an end-of-use indicator that signals to the user when to replace or refill the vapor-emitting device. Such an end-of-life indicator within a gel-based vapor-emitting device will give more reliable repurchase, and more confidence in using these repurchase figures in a business plan. Other desirable features and characteristics of the present invention will become apparent from the subsequent detailed description of the invention, the appended claims, and the accompanying drawing figures.

We describe herein various embodiments of a vapor-emitting device that includes an end-of-use indicator.

In one embodiment of the present invention, a vapor-emitting device is provided that comprises a housing including a cover and a base, a vertical support member, a gel matrix and an end-of-use indicator within the housing. The cover is displaceable along a vertical axis, relative to the base. The vertical support member is coupled to the base. The gel material includes a volatizable ingredient formulated therein and is formed about the vertical support. The gel material is configured to evaporate when the cover is displaced relative to the base, thereby exposing the gel material to an external environment. The end-of-use indicator is coupled to at least a portion of the housing and configured to alert a user that the gel material has evaporated to a pre-determined level. The end-of-use indicator is comprised of an indicia strip positioned within the gel material at an angle relative to the vertical axis of the device. The end-of life indicator may include at least one (1) raised indicia on a surface of the indicia strip that is visible when the gel material has evaporated to a pre-determined level.

In a further embodiment and still by way of example only, there is provided a vapor-emitting device comprised of a substantially cone-shaped housing including a cover and a base, a gel material including a volatizable ingredient contained within the housing, and an end-of-use indicator coupled to the base and configured to alert a user that the gel material has evaporated to a pre-determined level. The cover is displaceable relative to the base along a vertical axis. The gel material is configured to evaporate when the cover is displaced relative to the base, thereby exposing the gel material to an ambient external environment. The end-of-use indicator is comprised of an indicia strip positioned within the gel material at an angle relative to the vertical axis of the vapor-emitting device and coupled to a portion of the housing. The indicia strip may include raised indicia integrally part of, and protruding from, a surface of the indicia strip. During use, the gel material evaporates, dries and shrinks, retreating off the indicia strip, exposing the at least one indicia to visually indicate that the gel material has reached the pre-determined level.

Other independent features and advantages of the improved vapor-emitting device will become apparent from the following detailed description, taken in conjunction with the accompanying drawings that illustrate, by way of example, the principles of the invention.

### BRIEF DESCRIPTION OF THE DRAWINGS

The present invention will hereinafter be described in conjunction with the following drawing figure, wherein like numerals denote like elements. Additional embodiments of the invention will become evident upon reviewing the non-limiting embodiments described in the specification in conjunction with the accompanying figures, wherein:
FIGURE 1 is an upper perspective view of an exemplary embodiment of a vapor-emitting device in accordance with the present invention including a gel-based vapor releasing material and end-of-use indicator buried therein;
FIGURE 2 is a partial cross-sectional side view taken along line '2-2' of FIG. 1 showing a portion of the vapor-emitting device including an end-of-use indicator according to the present invention;
FIGURE 3 is an upper perspective view of a portion of an embodiment of the vapor-emitting device of the present invention, including an end-of-use indicator anchored within the base portion of a housing;
FIGURE 4 is a partial cross-sectional side view of an embodiment of a vapor-emitting device of the present invention including an end-of-use indicator having been exposed to an external environment for a period of time causing partial evaporation and shrinkage; and
FIGURE 5 is an upper perspective view of an embodiment of the vapor-emitting device of the present invention including an end-of-use indicator having been exposed to an external environment for a period of time.

### DETAILED DESCRIPTION OF THE INVENTION

The following detailed description of the invention is merely exemplary in nature and is not intended to limit the invention or the application and uses of the invention. Furthermore, there is no intent to be bound by any theory presented in the preceding background of the invention or in the following detailed description of the invention. Additionally there are many materials that the end-of-use indicator may be made from. Although described herein as preferably molded from plastic, metals (such as hammered tin or copper), glass, ceramic, wood, and the like, are all anticipated materials of construction and may become preferred for various reasons such as cost, stability with the vapor-emitting matrix, ease of suspending within a cast gel-based matrix, the way in which the vapor-emitting mass pulls away from its surface, novelty (e.g. end-of-life insert that doubles as a "collectable"), and other functional interactions with particular formulations of vapor-emitting matrices.

The detailed description of exemplary embodiments of the invention herein makes reference to the accompanying figures, which show the exemplary embodiments by way of illustration and its best mode. Thus, the detailed description herein is presented for purposes of illustration only and not by way of limitation.

The invention includes a vapor-emitting device (e.g., air freshener, odor neutralizer, insect repellant, insecticide, and the like). In an exemplary embodiment, the vapor-emitting device is configured to alert a user that it is time, or it is nearing the time, to refill or replace the device. To alert the user, the vapor-emitting device includes an end-of-use indicator that becomes visible when a pre-determined level of a gel material has been reached in the device. In the most preferred embodiment, the end-of-use indicator is an indicia strip suspended and anchored in a gel-based vapor-emitting device wherein evaporation and shrinkage of the gelmass gradually exposes the end-of-life indicator to signal to the user various stages along the life of the product.

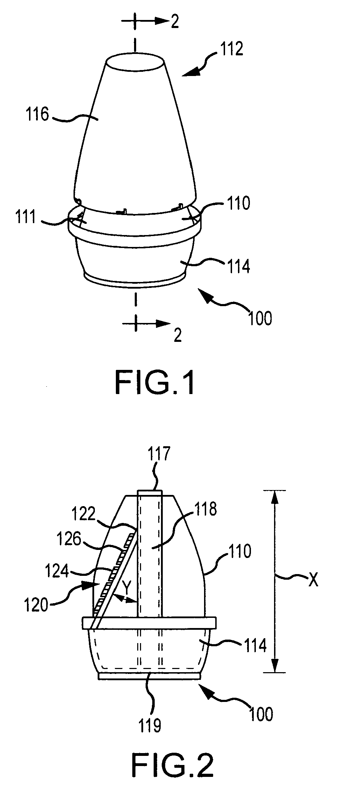

Turning now to the drawing figures, **FIGURE 1** is a diagram of an exemplary embodiment of a vapor-emitting device **100** including an end-of-use indicator that is not visible initially. Vapor-emitting device **100** may be any device and/or components known in the art capable of evaporating a volatizable ingredient (e.g., a fragrance, odor neutralizer, air sanitizer, medicinal agent, aroma therapeutic agent, insecticide, insect repellant, and the like). In this exemplary embodiment, vapor-emitting device **100** includes a gel material **110,** typically in the form of a solid or semi-solid mass, having an active or volatizable ingredient **111** contained therein. Vapor-emitting device **100** is preferably a "passive" vapor-emitting device, such that the gel material **110** containing volatizable ingredient **111** evaporates in ambient conditions into an environment that requires air treatment. In the most preferred embodiment, the gel material **110** is a cast matrix minimally comprising cellulose polymers such as carrageenan (a polysaccharide gellant), water, fragrance, dye and preservative. These cast gel-based materials are very well known in the art, especially in the context of passive gel-based air fresheners.

Vapor-emitting device **100** includes a housing **112** to contain the gel material **110,** and the housing comprises separate portions such as a base **114** and a movable cover **116.** Cover **116** is manually displaceable with respect to base **114.** More specifically, cover **116** is displaceable along a vertical axis of the vapor-emitting device **100** to control the effective rate of volatilization or evaporation of the gel material **110,** and more particularly the volatizable ingredient **111.** In the embodiment illustrated in **FIG 1****,** cover **116** is illustrated in a partially open position whereby the vapor-emitting gel material **110** is exposed to the environment to liberate the volatizable ingredient(s) **111.** Gel material **110** may include any volatizable ingredient **111** known in the art capable of, for example, altering the scent in an environment (e.g., a fragrance) and/or discouraging insects and the like from being present in the environment (e.g., an insecticide, an insect repellant, and the like). In this exemplary embodiment, gel material **110** is a carrageenan based gel that when molded around a surface will attach to that surface, and will pull back from the surface as it evaporates and/or vaporizes. Gel material **110,** including volatizable ingredient **111,** to which the present invention applies, are well known in the art, as are the methods of manufacture and the molding within a housing such as housing **112** shown in **FIG 1****.**

In this exemplary embodiment, vapor-emitting device **100,** and more particularly housing **112,** is generally formed as a cone-shaped device. It should be understood that although housing **112** is disclosed as being generally cone-shaped, alternative shapes for housing **112** are anticipated by this disclosure. In a preferred embodiment, base **114** and cover **116** are formed from injection-molded plastics, although other suitable materials are anticipated by this disclosure. It is important to realize that the housing **112** may be as simple as an open container (e.g., ajar with walls and bottom forming an interior) made from any suitable material such as glass, paperboard, wood, metal or plastic, inside of which the gel-based material **110** may be cast.

**FIGURE 2** is a partial cross-sectional view taken along line **`2-2'** of **FIG 1** of an exemplary embodiment of vapor-emitting device **100.** For purposes of illustration, **FIG 2** is a partial cross-section where cover **116** is not shown, gel material **110** is shown in cross section, and base **114** is shown in its entirety. In accordance with this exemplary embodiment, housing **112** includes therein a centrally located support member **118,** or a plurality of support members, around which the gel material **110,** including volatizable ingredient **111,** is molded or formed. Support member **118** provides vertical support and strength to gel material **110** housed within housing **112** and enables displacement of cover **116** (not shown). Support member **118** may be formed as an integral member with housing **112,** and more particularly integrally molded as part of the base **114,** or in the alternative, separately formed and positioned in place during assembly of housing **112.** Support member **118** is formed having a first end **117** and a second end **119,** separated by a length **x.** In this particular embodiment, second end **119** may be coupled to base **114** as illustrated, and in a preferred embodiment, second end **119** is contiguous and integrally molded with base **114** as a single injection-molded plastic part.

Vapor-emitting device **100** further includes an end-of-use indicator **120** to alert a user that housing **112** includes a low level of gel material **110,** and thus a low level of volatizable ingredient **111** or otherwise is in need of repurchase. In the exemplary embodiment, end-of-use indicator **120** is formed as an indicia strip **122,** generally in the shape of a substantially flat and thin strip of material that is physically coupled at one end to the base portion **114** or to the support member **118,** or at each of its ends, to both support member **118** and base **114.** Indicia strip **122** may include at least one visible mark or indicia **124,** preferably in the form of a raised element that protrudes from a viewable surface **126** of indicia strip **122.** In this particular embodiment illustrated, at least one visible mark **124** is in the form of a plurality of raised block-letter **"X"** shapes that form the visual end-of-use indicator **120** as described below. As mentioned, the end-of-use indicator **120** may be constructed out of a variety of materials. The preferred material is plastic, either injection molded plastic for a solid plastic end-of-use indicator, or molded from sintered plastic particles for a more porous plastic indicator. Preferred plastics include, but are not limited to, polyethylene, polypropylene, polyethylene terephthalate, nylon, polyvinyl chloride, polystyrene and the like. A molded plastic end-of-use indicator **120** made from any of these types of plastic will allow the gel material **110** to spread off the indicator as the gel material shrinks and dries up since most plastics are hydrophobic and the water-based gel matrix is necessarily hydrophilic. The surface of the end-of-use indicator **120** may be altered by a variety of means. For example, surface treatment of the plastic by chemical treatment and/or etching, texturing the surface or altering the height/shape of the raised markings (e.g., the **"X's" 124** on indicia strip **122**), such that the gel material **110** may initially grip, then retreat and pull away from the strip **122** at the desired rate. For example, judicious choice of material used to form the strip **122,** along with various surface treatments and/or judicial choice of embossed protrusions, will allow the gel material **110** to retreat off the strip **122** at a rate similar to its shrinkage around support **118.** In another embodiment, end-of-use indicator **120** may be a molded porous plastic piece. In this way the adherence of the gel material **110** to the strip **122** can be varied by the degree of porosity and/or the porous surface texture. Although somewhat empirical, the extent to which the strip **122** becomes visible over time as the gel material **110** dries and shrinks away from it can be fully customized.

It is important to realize that when constructed of plastic, end-of-use indicator **120,** and in particular indicia strip **122,** may be formed from a plastic that is different than that used for the housing **112.** As mentioned previously, the end-of-use indicator **120** may be constructed of other less preferred materials such as hammered tin or copper, or molded from glass or ceramics.

In an unusual embodiment, end-of-use indicator **120** may be a detachable souvenir of sorts, a "collectable" that the excited consumer sees appearing gradually, which then may be detached from the housing to save.

Indicia strip **122** is positioned relative to support member **118** at an angle dependent upon the design of the overall vapor-emitting device **100** and the evaporative properties of gel material **110.** More specifically, as best illustrated in **FIG 2****,** when gel material **110** is initially formed, indicia strip **122** may be positioned within gel **material 110** and at an angle **Y** relative to support member **118,** to allow for complete initial coverage of the indicia **124,** and to adjust the rate of retreat by gel material **110** off the strip **122.**

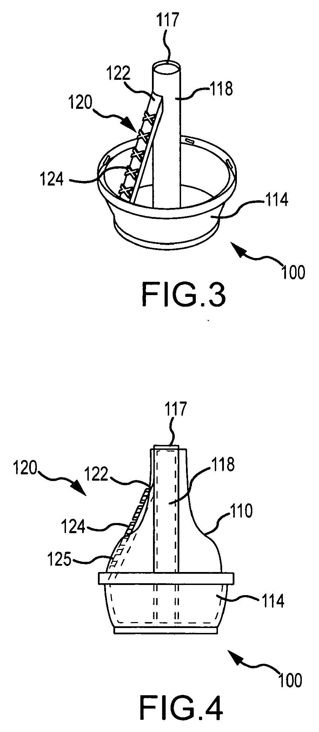

**FIGURE 3** illustrates a preferred embodiment for the vapor-emitting device of the present invention wherein the end-of-use indicator **120** is an indicia strip **122** angled between the support member **118** and base portion **114** of housing **112.** As mentioned, the strip **122** may be physically anchored to either the support member **118,** to the base portion **114,** or to both of these housing portions. Further illustrated in **FIG 3** is the preferred embodiment for the indicia strip **122** wherein raised marks such as block letter **"X's" 124** are incorporated on the surface of **122** most viewable by the user. As mentioned, the number/height/size of the raised indicia **124** are adjusted to regulate the rate at which the gel material **110** retreats off the angled strip **122** as it dries and shrinks, and to provide visible indication to the user such as in the form of words or letters (e.g., "X", "REPLACE", "END", or "BUY MORE"). In this way design and novelty elements may be merged with the functional aspects for the raised indicia **124.**

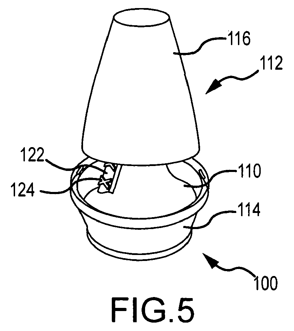

**FIGURES 4** **and** **5** illustrate in partial cross-section and in isometric view, respectively, an exemplary embodiment of the vapor-emitting device **100** after exposure to an external environment for a period of time. As mentioned, the gel material **110** necessarily dries out and shrinks as its volatile constituents, (including such ingredients as water, air treatment materials, and volatile solvents), evaporate. **FIGS 4** **and** **5** illustrate gel material **110** in a partially evaporated and/or vaporized state. More particularly, as gel material **110** evaporates and necessarily dries and shrinks, indicia strip **122** becomes increasingly more exposed, beginning first at the mid section and end of strip **122** that is positioned at the support member **118.** It should be noted that gel material **110** undergoes a reliable geometric/conformational change during use that is dependent upon the shape of the gel mass and the allowed air flow to it. Accordingly, the indicia strip **122** is positionable in reliance upon this geometric/conformational change. More particularly, the indicia strip **122** may be angled and anchored within the cone-shaped gel material **110** so that as the gel material **110** dries and shrinks from a conical shape to more of a "pear shape", the indicia strip **122** gradually becomes visible in a predictable and reproducible manner. As illustrated, during evaporation and/or vaporization of gel material **110,** the at least one indicia **124** becomes exposed and visually revealed to the user monitoring the use-up of the product. The indicia **124** may be strategically positioned along the indicia strip **122** such that the user will see the indicia **124** when the volatizable ingredient is gone, not at the point when the gel material is entirely dried up and shrunk into a small mass. The reveal of indicia **124** on indicia strip **122** may be designed to provide an indication of the intensity of the remaining fragrance, etc., and/or a time period remaining prior to the depletion of gel material **110.** For instance, during use of vapor-emitting device **100,** gel material **110** gradually evaporates, binding across and stretching away from indicia strip **122.** In the illustrated exemplary embodiment in **FIGS 4** **and** **5****,** indicia strip **122** includes a plurality of visual indicia **124** in the form of raised block letter **X's** along the length of indicia strip **122.** When gel material **110** begins to bind across indicia strip **122,** a first **X** indicia **124** will be exposed. This may provide a visual indication to the user of a remaining time period in which gel material **110** will be present, or a visual indicator as to the remaining intensity of volatizable ingredient **111** contained in gel material **110.** As subsequent indicia **124,** or more **X's,** are exposed, the user may become visually aware of the time period remaining, or the intensity of volatizable ingredient **111.**

In this way, linking a visual queue to the point in time when the device has outlived its usefulness as an air treatment product can accelerate the repurchase rate of the product. As illustrated in **FIG 5****,** end-of-use indicia **124** may become visible to the user long before the gel matrix **110** has fully shrunk, thus telling the consumer to repurchase at the time when the product no longer functions, and ensuring that the consumer won't wait until the gel mass is entirely dried out before repurchase.

It should be understood that while indicia **124** in this exemplary embodiment are in the form of raised block letter **X's,** any type of indicia **124** that is visible from the surface of indicia strip **122** or otherwise embossed or recessed, provides a binding surface for the gel material **110** and may be used. More particularly, it is anticipated that letters and/or numbers may be used to visibly indicate information to the user. For example, in another exemplary embodiment, end-of-use indicator **120,** and more specifically indicia strip **122** may have formed on its visible surface a series of raised letters and/or numbers indicating for example a "four weeks", "three weeks" "two weeks", "one week", and so forth to indicate the remaining useful life of gel material **110,**

Accordingly, we have disclosed an improved vapor-emitting device that includes a visual end-of-use indicator. While exemplary embodiments have been presented in the foregoing detailed description of the invention, it should be appreciated that a vast number of variations exist. In addition, benefits, other advantages, and solutions to the problem have been described herein with regard to exemplary embodiments. However, the benefits, advantages, solutions to problems, and any element(s) that may cause any benefit, advantage, or solution to occur or become more pronounced are not to be construed as critical, required, or essential features or elements of any or all the claims or the invention. It should also be appreciated that the exemplary embodiments are only examples, and are not intended to limit the scope, applicability, or configuration of the invention in any way.

## Claims

1. A vapor-emitting device comprising; a. a housing comprising a cover and a base, wherein the cover is displaceable along a vertical axis relative to said base; b. a vertical support member with first and second ends, one of said ends coupled to said base; c. a gel material including a volatizable ingredient dispersed therein, the gel material formed about the vertical support and configured to evaporate when the cover is displaced relative to the base exposing the gel material to an external environment; and, d. an end-of-use indicator coupled to the housing and configured to alert a user that the gel material has evaporated to a pre-determined level; said end-of-use indicator comprising;
(i) an indicia strip positioned within the gel material at an angle relative to the vertical axis; and, (ii) at least one indicia on a surface of the indicia strip and visible when the gel material has evaporated to the pre-determined level.

2. The vapor-emitting device of Claim 1, wherein the volatizable ingredient is selected from the group consisting of fragrances, odor neutralizers, air sanitizers, insecticides, and insect repellants, and mixtures thereof.

3. The vapor-emitting device of Claim 1, wherein the indicia strip is positioned within the gel material at an angle relative to the vertical axis of the vapor-emitting device and coupled to the base.

4. The vapor-emitting device of Claim 1, wherein the indicia strip is positioned within the gel material at an angle relative to the vertical axis of the vapor-emitting device and is coupled to the vertical support member.

5. The vapor-emitting device of Claim 1, wherein the housing is substantially coneshaped.

6. The vapor-emitting device of Claim 1, wherein the at least one indicia includes a plurality of indicia configured to become visible as said gel material evaporates.

7. The vapor-emitting device of Claim 1, wherein the at least one indicia includes a plurality of indicia configured to indicate the remaining volatizable ingredient as the said indicia become visible.

8. A vapor-emitting device comprising; a. a substantially cone-shaped housing comprising a cover and a base, wherein the cover is displaceable relative to the base along a vertical axis; b. a gel material including a volatizable ingredient contained therein the housing, said gel material configured to evaporate when the cover is displaced relative to the base, thereby exposing the gel material to an external environment; and, c. an end-of-use indicator coupled to said base and configured to alert a user that the gel material has evaporated to a predetermined level, said end-of-use indicator comprising;
(i) an indicia strip positioned within the gel material at an angle relative to the vertical axis of the vapor-emitting device; and, (ii) at least one indicia formed on a surface of the indicia strip, wherein said gel material evaporates over time and retreats off the indicia strip, exposing the at least one indicia and visually indicating the gel material has reached a pre-determined level.

9. The vapor-emitting device of Claim 8, further including a vertical support member coupled to the base, the gel material formed about said vertical support member

10. The vapor-emitting device of Claim 9, wherein the indicia strip is further coupled to the vertical support member.

## Patentansprüche

1. Dampfausstoßvorrichtung, Folgendes umfassend:
a. ein Gehäuse, umfassend eine Abdeckung und eine Basis, wobei die Abdeckung entlang einer vertikalen Achse bezogen auf die Basis verschiebbar ist;
b. ein vertikales Stützelement mit einem ersten und einem zweiten Ende, wobei eines der Enden mit der Basis gekoppelt ist;
c. ein Gelmaterial, enthaltend einen darin dispergierten verflüchtigbaren Inhaltsstoff, wobei das Gelmaterial um die vertikale Stütze herum ausgebildet ist und konfiguriert ist, zu verdampfen, wenn die Abdeckung bezogen auf die Basis verschoben wird, was das Gelmaterial einer externen Umgebung aussetzt; und
d. eine Anzeige des Verwendungsendes, die mit dem Gehäuse gekoppelt ist und konfiguriert ist, einen Benutzer zu warnen, dass das Gelmaterial auf einen vorgegebenen Stand verdampft ist; wobei die Anzeige des Verwendungsendes Folgendes umfasst:
(i) einen Markierungsstreifen, der im Gelmaterial in einem Winkel bezogen auf die vertikale Achse positioniert ist; und
(ii) wenigstens eine Markierung auf einer Oberfläche des Markierungsstreifens, und die sichtbar ist, wenn das Gelmaterial auf den vorgegebenen Stand verdampft ist.

2. Dampfausstoßvorrichtung nach Anspruch 1, wobei der verflüchtigbare Inhaltsstoff ausgewählt ist aus der Gruppe bestehend aus Duftstoffen, Geruchsneutralisationsmitteln, Luftreinigungsmitteln, Insektiziden und Insektenschutzmitteln und Mischungen daraus.

3. Dampfausstoßvorrichtung nach Anspruch 1, wobei der Markierungsstreifen im Gelmaterial in einem Winkel bezogen auf die vertikale Achse der Dampfausstoßvorrichtung positioniert ist und mit der Basis gekoppelt ist.

4. Dampfausstoßvorrichtung nach Anspruch 1, wobei der Markierungsstreifen im Gelmaterial in einem Winkel bezogen auf die vertikale Achse der Dampfausstoßvorrichtung positioniert ist und mit dem vertikalen Stützelement gekoppelt ist.

5. Dampfausstoßvorrichtung nach Anspruch 1, wobei das Gehäuse im Wesentlichen kegelförmig ist.

6. Dampfausstoßvorrichtung nach Anspruch 1, wobei die wenigstens eine Markierung mehrere Markierungen enthält, die konfiguriert sind, sichtbar zu werden, wenn das Gelmaterial verdampft.

7. Dampfausstoßvorrichtung nach Anspruch 1, wobei die wenigstens eine Markierung mehrere Markierungen enthält, die konfiguriert sind, den verbleibenden verflüchtigbaren Inhaltsstoff anzuzeigen, während die Markierungen sichtbar werden.

8. Dampfausstoßvorrichtung, Folgendes umfassend:
a. ein im Wesentlichen kegelförmiges Gehäuse, das eine Abdeckung und eine Basis umfasst, wobei die Abdeckung bezogen auf die Basis entlang einer vertikalen Achse verschiebbar ist;
b. ein Gelmaterial, das einen darin im Gehäuse enthaltenen verflüchtigbaren Inhaltsstoff enthält, wobei das Gelmaterial konfiguriert ist, zu verdampfen, wenn die Abdeckung bezogen auf die Basis verschoben wird, dadurch Aussetzen des Gelmaterials einer externen Umgebung; und
c. eine Anzeige des Verwendungsendes, die mit der Basis gekoppelt ist und konfiguriert ist, einen Benutzer zu warnen, dass das Gelmaterial auf einen vorgegebenen Stand verdampft ist, wobei die Anzeige des Verwendungsendes Folgendes umfasst:
(i) einen Markierungsstreifen, der im Gelmaterial in einem Winkel bezogen auf die vertikale Achse der Dampfausstoßvorrichtung positioniert ist; und
(ii) wenigstens eine Markierung, die auf einer Oberfläche des Markierungsstreifen ausgebildet ist, wobei das Gelmaterial im Laufe der Zeit verdampft und sich vom Markierungsstreifen zurückzieht, was die wenigstens eine Markierung freilegt und visuell anzeigt, dass das Gelmaterial einen vorgegebenen Stand erreicht hat.

9. Dampfausstoßvorrichtung nach Anspruch 8, ferner enthaltend ein vertikales Stützelement, das mit der Basis gekoppelt ist, wobei das Gelmaterial um das vertikale Stützelement herum ausgebildet ist.

10. Dampfausstoßvorrichtung nach Anspruch 9, wobei der Markierungsstreifen ferner mit dem vertikalen Stützelement gekoppelt ist.

## Revendications

1. Dispositif émetteur de vapeurs comprenant :
a. un logement comprenant un couvercle et une base, le couvercle étant déplaçable le long d'un axe vertical par rapport à ladite base ;
b. un élément support vertical avec une première et une deuxième extrémités, une desdites extrémités étant couplée à ladite base ;
c. un matériau en gel incluant un ingrédient volatilisable dispersé dedans, le matériau en gel étant formé autour du support vertical et conçu pour s'évaporer quand le couvercle est déplacé par rapport à la base, exposant le matériau en gel à un environnement externe ; et
d. un indicateur de fin d'utilisation couplé au logement et conçu pour alerter un utilisateur que le gel s'est évaporé jusqu'à un niveau prédéterminé, ledit indicateur de fin d'utilisation comprenant :
i) une bande de marquage positionnée à l'intérieur du matériau en gel à un angle donné par rapport à l'axe vertical ; et
ii) au moins un marquage sur une surface de la bande de marquage et visible quand le matériau en gel s'est évaporé à un niveau prédéterminé.

2. Dispositif émetteur de vapeurs selon la revendication 1, dans lequel l'ingrédient volatilisable est choisi dans le groupe constitué de parfums, neutraliseurs d'odeurs, purificateurs d'air, insecticides, répulsifs pour insectes et leurs mélanges.

3. Dispositif émetteur de vapeurs selon la revendication 1, dans lequel la bande de marquage est positionnée à l'intérieur du matériau en gel à un angle donné par rapport à l'axe vertical du dispositif émetteur de vapeurs et est couplée à la base.

4. Dispositif émetteur de vapeurs selon la revendication 1, dans lequel la bande de marquage est positionnée à l'intérieur du matériau en gel à un angle donné par rapport à l'axe vertical du dispositif émetteur de vapeurs et est couplée à l'élément support vertical.

5. Dispositif émetteur de vapeurs selon la revendication 1, dans lequel le logement a essentiellement la forme d'un cône.

6. Dispositif émetteur de vapeurs selon la revendication 1, dans lequel l'au moins un marquage inclut une pluralité de marquages conçus pour devenir visibles à mesure que ledit matériau en gel s'évapore.

7. Dispositif émetteur de vapeurs selon la revendication 1, dans lequel l'au moins un marquage inclut une pluralité de marquages conçus pour indiquer le reste d'ingrédient volatilisable à mesure que lesdits marquages deviennent visibles.

8. Dispositif émetteur de vapeurs comprenant :
a. un logement de forme essentiellement conique comprenant un couvercle et une base, le couvercle étant déplaçable le long d'un axe vertical par rapport à ladite base ;
b. un matériau en gel incluant un ingrédient volatilisable dispersé dedans, le matériau en gel étant conçu pour s'évaporer quand le couvercle est déplacé par rapport à la base, exposant le matériau en gel à un environnement externe ; et
c. un indicateur de fin d'utilisation couplé à ladite base et conçu pour alerter un utilisateur que le matériau en gel s'est évaporé à un niveau prédéterminé, ledit indicateur de fin d'utilisation comprenant :
i) une bande de marquage positionnée à l'intérieur du matériau en gel à un angle donné par rapport à l'axe vertical du dispositif émetteur de vapeurs ; et
ii) au moins un marquage formé sur une surface de la bande de marquage,
ledit matériau en gel s'évaporant avec le temps et se retirant de la bande de marquage, exposant l'au moins un marquage et indiquant visuellement que le matériau en gel a atteint un niveau prédéterminé.

9. Dispositif émetteur de vapeurs selon la revendication 8, incluant en outre un élément support vertical couplé à la base, le matériau en gel étant formé autour dudit élément support vertical.

10. Dispositif émetteur de vapeurs selon la revendication 9, dans lequel la bande de marquage est, en outre, couplée à l'élément support vertical.
